# EUROPEAN PATENT APPLICATION

(11) **EP 4 546 358 A1**
(43) Date of publication of application: **30.04.2025**
(21) Application number: 24208185.9
(22) Date of filing: 22.10.2024
(51) Int. Cl.: G16H 20/10, G16H 20/30, G16H 20/60, G16H 40/63

(54) **INFORMATION INPUT SUPPORT SYSTEM AND INFORMATION INPUT SUPPORT PROGRAM**

(30) Priority: 24.10.2023 JP 2023182771
(71) Applicant: ARKRAY, Inc., Kyoto-shi, Kyoto 601-8045 (JP)
(72) Inventor: KAI, Akinori, Kyoto, 602-0008 (JP)
(74) Representative: Dehns

(57) **Abstract**

An information input support system (100) allows text generative Al to generate guidance comments on a management item for a target person to be managed. The information input support system includes a data acquisition unit (10), a command creation unit (20), and an instruction unit (80). The data acquisition unit (10) includes a target person information acquisition portion (12) that acquires target person data belonging to the target person to be managed, and a guidance information acquisition portion (13) that acquires guidance item data indicating the content of guidance that is offered in the guidance comments. The command creation unit (20) creates instruction commands that instruct the text generative Al to generate text based on the target person data and the guidance item data that have been acquired by the data acquisition unit (10). The instruction unit (80) provides the text generative Al with the instruction commands created by the command creation unit (20).

## Description

### BACKGROUND OF THE INVENTION

### 1. Field of the Invention

The present invention relates to an information input support system and an information input support program that are used to provide guidance comments to target people to be managed such as patients and those carrying out health care programs in the medical and health care fields. In particular, the present invention relates to an information input support system and an information input support program that employ text generative artificial intelligence (AI) to generate guidance comments, and that create instruction commands that provide the text generative AI with text generation requirements to obtain desired guidance comments from the text generative AI.

### 2. Description of Related Art

Physicians and health care advisers (also referred to as a manager/managers in the following) have regularly provided lifestyle guidance to their patients with lifestyle-related diseases such as diabetes and those participating in health care programs (also referred to as a target person/target people to be managed in the following) in order to treat the diseases and promote health.

The lifestyle guidance is intended, e.g., to help the target people to be managed deal with challenges they may face on a daily basis and to offer praise and encouragement when they are successful in overcoming the challenges in a certain period of time. The managers usually provide their comments in written form and encourage the target people to better understand and be more involved in their health care issues. These efforts are considered to yield significant results.

However, writing comments on different health problems and outcomes for individuals constitutes a great burden on the managers.

Japanese Patent No. 6261548 proposes a health management server that assists health professionals in preparing and sending advice messages to their clients in a conventional health care program that manages the health care of the clients. The health management server previously stores various illustrations and example sentences, from which the health professionals select suitable ones for the clients concerned. Then, the server prepares advice messages based on the selection results and transmits the advice messages along with the selected illustrations to the clients' terminals.

JP 2004-157815 A proposes a medical image report input system that creates diagnostic comments on medical images, in which the diagnostic comments and the medical images are stored together. The system uses templates to retain the display order of the portions of the medical images to be interpreted and their response candidates. Physicians select the medical images and the response candidates in accordance with the templates. Thus, the system can combine the selected response candidates into diagnostic comments.

The conventional writing support technology allows two or more example sentences to be combined to create the advice messages or diagnostic comments as described above, and therefore can significantly reduce the burden on the managers such as physicians and health care advisers, compared to the case where they have to create text from the beginning.

However, simply combining predetermined sentences may result in a lack of contextual connection between the sentences. Consequently, the entire text tends to be more unnatural and unsatisfactory than the text written by the managers themselves.

On the other hand, text generative AI has rapidly evolved and become more readily available in recent years. The text generative AI, which is called chatbot, is designed to generate text that resembles human conversation or the like. Since the text generated by text generative AI flows smoothly and appears natural, if text generative AI is use to generate guidance comments that are sent from the managers to the target people to be managed, it is expected that the AI-generated comments will be natural and acceptable to the target people without any discomfort. For this purpose, the input commands given to the text generative AI must have necessary and sufficient information so that the AI can generate accurate and natural-sounding guidance comments for each situation.

To solve the conventional problems, embodiments of the present invention provide an information input support system and an information input support program that create instruction commands that instruct text generative AI to generate guidance comments that feel more natural and contain necessary information.

In other words, embodiments of the present invention can easily provide guidance comments that are written in a natural style and contain necessary information by using text generative AI.

### GENERAL DESCRIPTION

An information input support system of the present invention allows text generative AI to generate guidance comments on a management item for a target person to be managed. The information input support system includes a data acquisition unit, a command creation unit, and an instruction unit. The data acquisition unit includes a target person information acquisition portion that acquires target person data belonging to the target person to be managed, and a guidance information acquisition portion that acquires guidance item data indicating the content of guidance that is offered in the guidance comments. The command creation unit creates instruction commands that instruct the text generative AI to generate text based on the target person data and the guidance item data that have been acquired by the data acquisition unit. The instruction unit provides the text generative AI with the instruction commands created by the command creation unit.

With this configuration, the information input support system of the present invention makes full use of the advantages of the text generative AI capable of generating text in a natural style, and thus can easily create guidance comments that are easy to understand and contain necessary information.

Optionally, the information input support system further includes a storage unit that stores data concerning a plurality of guidance items that has been previously prepared. Optionally, the guidance information acquisition portion receives a selection of at least one guidance item from the previously prepared guidance items and acquires data concerning the at least one guidance item. This correlates the content of guidance offered in the guidance comments with each guidance item, and helps the target person to be managed have a deeper understanding of the guidance items.

Optionally, the target person data has a plurality of items, and the storage unit stores a set of options that has been previously prepared for a predetermined item of the plurality of items of the target person data. Optionally, the target person information acquisition portion receives a selection of an option from the set of options and acquires the target person data corresponding to the predetermined item. This facilitates the entry of the target person data and reduces the burden on the manager.

Moreover, the target person information acquisition portion optionally acquires the target person data from a target person information obtaining means for measuring or managing target person data belonging to the target person to be managed. This saves the manager from having to enter the target person data that has already been acquired by the target person information obtaining means, which is optionally an external device.

Optionally, the data acquisition unit further includes a text information acquisition portion that acquires text information data indicating formal aspects of the guidance comments to be generated by the text generative AI, and the command creation unit creates the instruction commands based on the text information data. This ensures that the AI-generated guidance comments become more desirable.

Optionally, the command creation unit creates the instruction commands with priority given to the guidance item data acquired by the guidance information acquisition portion of the data acquired by the data acquisition unit. This establishes a close correlation between the content of guidance offered in the guidance comments and the management item.

Optionally, the target person to be managed may be a diabetic patient and the management item may be diabetes.

An information input support program of the present invention allows text generative AI to generate guidance comments on a management item for a target person to be managed. The information input support program includes creating instruction commands that instruct the text generative AI to generate the guidance comments based on target person data belonging to the target person to be managed and guidance item data indicating the content of guidance that is offered in the guidance comments, both the target person data and the guidance item data having been acquired by a data acquisition unit.

With this configuration, the information input support program of the present invention makes full use of the advantages of the text generative AI capable of generating text in a natural style, and thus can easily create guidance comments that feel natural and contain necessary information.

Hereinafter, embodiments of the information input support system and the information input support program of the present invention will be described with reference to the drawings.

In the following embodiments, assuming that the target person to be managed is a diabetic patient and the manager is a physician in charge of providing lifestyle guidance to the diabetic patient for medical care in diabetes, text generative AI is used to generate guidance comments that are presented along with a guidance item card with appropriate timing.

### BRIEF DESCRIPTION OF THE DRAWINGS

FIG. 1 is a block diagram illustrating the configuration of an information input support system of an embodiment of the present invention.
FIG. 2 is a flow chart for an information input support program of an embodiment of the present invention.
FIG. 3 is a diagram illustrating an example of an information input screen for supporting the entry of data that is acquired by a target person information acquisition portion and a text information acquisition portion.
FIG. 4 is a diagram illustrating an example of an information input screen for supporting the entry of data that is acquired by a guidance information acquisition portion.
FIG. 5 is a diagram illustrating an example of a medical care guidance card issued by the Japan Association for Diabetes Education and Care (JADEC).

### DETAILED DESCRIPTION

### (Embodiments)

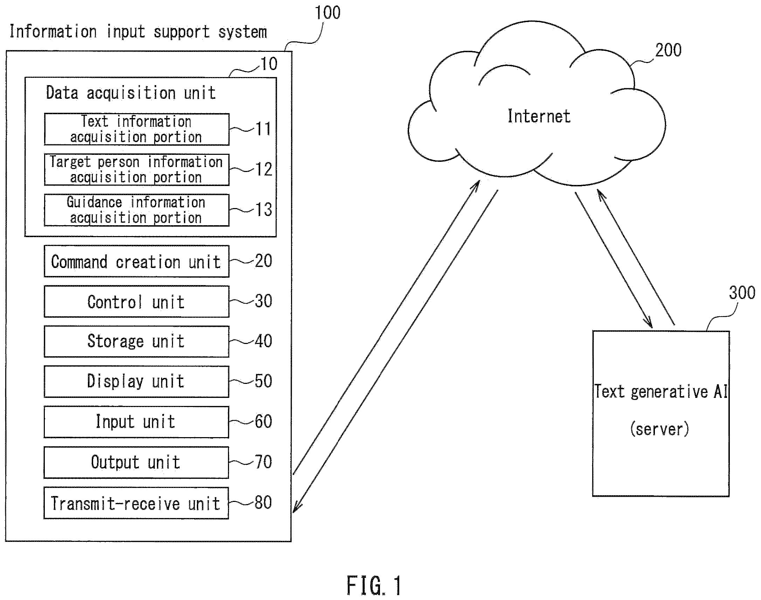

FIG. 1 is a block diagram illustrating the overall configuration of an information input support system according to an embodiment of the present invention.

In FIG. 1, the components of the information input support system are represented in the form of blocks from a functional viewpoint rather than a physical viewpoint. Therefore, in some cases, one block may represent a plurality of electronic components. In other cases, a plurality of blocks may represent a circuit mounted on a single circuit board.

As illustrated in FIG. 1, an information input support system 100 of this embodiment includes a data acquisition unit 10, a command creation unit 20, a control unit 30, a storage unit 40, a display unit 50, an input unit 60, an output unit 70, and a transmit-receive unit (instruction unit) 80.

The data acquisition unit 10 acquires a variety of information used to create instruction commands that instruct text generative AI to generate desired guidance comments. The data acquisition unit 10 includes a text information acquisition portion 11, a target person information acquisition portion 12, and a guidance information acquisition portion 13. The text information acquisition portion 11 acquires text information data indicating formal aspects (e.g., wording, style, content, etc.) of the text in the guidance comments. The target person information acquisition portion 12 acquires target person data belonging to the target person to be managed such as a diabetic patient who receives the guidance comments. The guidance information acquisition portion 13 acquires guidance item data indicating the content of guidance that is offered in the guidance comments.

Specifically, the text information data acquired by the text information acquisition portion 11 indicates formal aspects of the guidance comments such as one or more of the type of language, the total number of characters, the tone of writing, and the option of whether or not to include greetings and self-introductions.

The target person data acquired by the target person information acquisition portion 12 indicates a variety of information that is effective in making the guidance comments more specific and in line with the target person's needs. Examples of the information include: essential information for identifying the target person who receives the guidance comments, such as one or more of: full name and identification number (ID); biometric information of the target person; and information about the current condition of the target person participating in the health care program.

The guidance item data acquired by the guidance information acquisition portion 13 indicates information concerning the guidance items under which the details of guidance such as example sentences that may be used in the guidance comments are classified, and the specific contents of these guidance items.

The information input support system 100 assists the physician in charge of providing the guidance comments to easily and reliably enter the text information data, the target person data, and the guidance item data, which will be acquired by the data acquisition unit 10, by presenting a set of options for each input item or obtaining necessary information from an external device or application.

More specifically, the information input support system 100 displays an information input screen on a display screen of the display unit 50, as will be described later. The information input screen enables the physician to identify a diabetic patient by entering the patient's full name and/or ID, and subsequently to choose any of the options listed for each item in the target person data, e.g., the patient's characteristics and/or biometric information, and also to choose any of the options listed for each item in the text information data, e.g., one or more of the language, character count, and tone of writing of the guidance comments.

Further, data entry by the physician using the information input screen can be omitted in the following manner. For example, the data acquisition unit 10 may read information that identifies the target person to be managed by linking the input unit 60 to the information input screen, as will be described later. Alternatively, the data acquisition unit 10 may receive the target person data (e.g., biometric information, current condition, etc.) of the target person to be managed from a target person information obtaining means (not shown) such as an external measuring device or a portable terminal for operating a management application. In this case, the target person information obtaining means has previously obtained the information concerning the biometric information, the current condition, etc. of the target person to be managed.

Furthermore, the target person data and the previous instruction commands are associated with the corresponding target person, and then stored in the storage unit 40, as will be described later. When the target person is identified, the data stored in the storage unit 40 may be retrieved and displayed on the display unit 50 or displayed by default as the selected items on the information input screen.

The command creation unit 20 creates instruction commands that instruct the text generative AI on what guidance comments to generate based on the target person data, the guidance item data, and the text information data that have been acquired by the data acquisition unit 10.

The instruction commands created by the command creation unit 20 follow the prompt format of the text generative AI and give the AI necessary and sufficient information to achieve desired guidance comments.

Among the target person data, the guidance item data, and the text information data that have been acquired by the data acquisition unit 10, the command creation unit 20 focuses on the guidance item data of the guidance information acquisition portion 13 and creates instruction commands so that the whole of the guidance item data is reflected in the guidance comments to be generated by the text generative AI. Therefore, the instruction commands are given to the text generative AI to generate text that appropriately reflects the content of guidance.

The control unit 30 controls the overall operation of the information input support system 100.

The control unit 30 may be composed of a known microprocessor or electronic logic circuit. When the information input support system 100 is used as software for a personal computer or as an application for a smartphone or a tablet, the central processing unit (CPU) of the personal computer, the tablet, or the smartphone acts as the control unit 30.

The storage unit 40 stores, e.g., the following: the target person data, the guidance item data, and the text information data that have been acquired by the data acquisition unit 10; some of the target person data and some of the text information data, which are associated with the target person to be managed and presented by the data acquisition unit 10 as the input support information to facilitate data entry by the physician; the instruction commands created by the command creation unit 20; and a program for operating the information input support system 100.

The storage unit 40 also stores the guidance comments generated by the text generative AI along with the evaluation of the AI-generated guidance comments and the instruction commands used to generate these guidance comments. Thus, the instruction commands can be analyzed based on the evaluation of the AI-generated guidance comments. Such analysis is useful in creating better instruction commands.

The storage unit 40 may be composed of various known memory circuits. Like the control unit 30, when the information input support system 100 is used as software for a personal computer or as an application for a smartphone or a tablet, the memory of each of the electronic devices acts as the storage unit 40.

The display unit 50 includes a display device (display terminal) having a display screen such as a liquid crystal panel or an electroluminescent (EL) panel, and a control portion for displaying a desired image on the display device. The display unit 50 displays, e.g., various types of information (data) acquired by the data acquisition unit 10 and the information input screen that assists the manager to enter necessary information into the data acquisition unit 10. The display unit 50 also displays the created instruction commands and the AI-generated guidance comments, and thus allows the manager (user) to confirm their respective contents.

When the information input support system 100 is used as software for a personal computer or as an application for a smartphone or a tablet, the display screen and the image display driver of each of the electronic devices act as the display unit 50.

The information input support system 100 receives data from the target person information obtaining means through the input unit 60. Examples of the target person information obtaining means include an external measuring device such as a weight scale or a body composition monitor and a portable terminal such as a smartphone or a tablet, on which software or an application is installed to operate other health care programs.

The input unit 60 can be used as a data input unit that is connectable to the target person information obtaining means (external device) and also as a data reading unit that reads necessary information from a storage medium such as a USB memory. When the input unit 60 functions as a bar code reader that reads a one-dimensional or two-dimensional bar code, the input unit 60 can read information that identifies the target person to be managed from a bar code provided on, e.g., the patient ID card or medical record of the target person such as a diabetic patient. Moreover, when the input unit 60 can transmit/receive data to/from the target person information obtaining means via a short-distance communication means such as Bluetooth (registered trademark), the input unit 60 functions as a unit for acquiring necessary information from the communication data received by the transmit-receive unit 80, as will be described later.

The output unit 70 outputs various types of data, including the instruction commands, the AI-generated guidance comments, and a list of information on the target person to be managed, from the information input support system 100 to the outside.

The output unit 70 with a print function can directly print the output data. Further, the output unit 70 can have an output function such that data is converted into an appropriate data format and stored in various storage media such as a USB memory, e.g., when printing the data by a printer of another device, displaying the data on a display monitor of another device, or saving the data to an external device.

The transmit-receive unit 80 has a transmitting function to transmit data from the information input support system 100 to an external device and a receiving function to receive data from the external device.

In the most general form of the information input support system 100, the instruction commands created by the command creation unit 20 are transmitted by the transmitting function of the transmit-receive unit 80. Then, the text generative AI incorporated in an external server 300 receives the instruction commands through the Internet environment 200, as illustrated in FIG. 1, and generates guidance comments according to the instruction commands. Therefore, in the information input support system 100, the transmit-receive unit 80 corresponds to the instruction unit that instructs the text generative AI to generate guidance comments. If instead the text generative AI is installed as software on an information device such as a personal computer that the manager would use, an information transmission unit will transmit the instruction commands to the text generative AI present in the same information device and instruct the AI to generate guidance comments. In this case, the information transmission unit corresponds to the instruction unit.

The guidance comments generated by the text generative AI are transmitted from the server 300 and received by the transmit-receive unit 80 of the information input support system 100 through the Internet environment 200. Thus, the physician can check the AI-generated guidance comments on the display terminal of the display unit 50. The AI-generated guidance comments can be stored in the storage unit 40, as described above.

The transmit-receive unit 80 may use various carrier communications under the Internet environment 200. When the data acquisition unit 10 receives the target person information from different management software or applications, or it receives the measurement results from biometric measuring devices such as a weight scale and/or a blood-pressure meter, the transmit-receive unit 80 preferably supports multiple communication means so that a short-distance communication means such as Bluetooth (registered trademark) can be used to communicate with the target person information obtaining means (external device).

The following is a detailed explanation of the procedure for providing the guidance comments to a diabetic patient by the physician in charge with the use of an information input support program that is run on the information input support system of this embodiment.

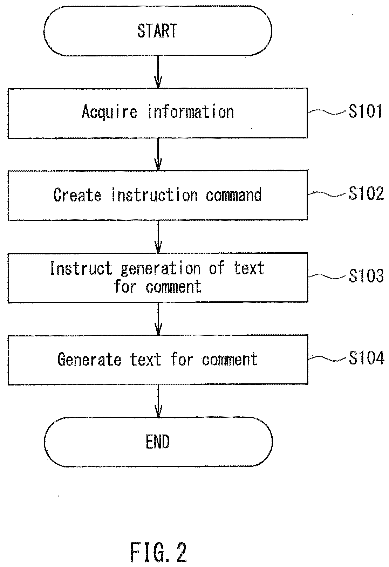

FIG. 2 is a flow chart illustrating the operation of an information input support program of this embodiment.

As illustrated in FIG. 2, the information input support program of this embodiment includes the following steps: acquiring information about the target person to be managed and the content of guidance (i.e., the target person data, the guidance item data, and the text information data) by the data acquisition unit 10 of the information input support system 100 (Step S101); creating instruction commands by the command creation unit 20 based on the information (the target person data, the guidance item data, and the text information data) acquired by the data acquisition unit 10 (Step S102); instructing the generation of comment text upon transmission of the instruction commands to the server 300 equipped with the text generative AI by the transmit-receive unit 80 serving as the instruction unit (Step S103); and providing the text generative AI with the instruction commands to generate guidance comments according to the instruction commands (Step S104).

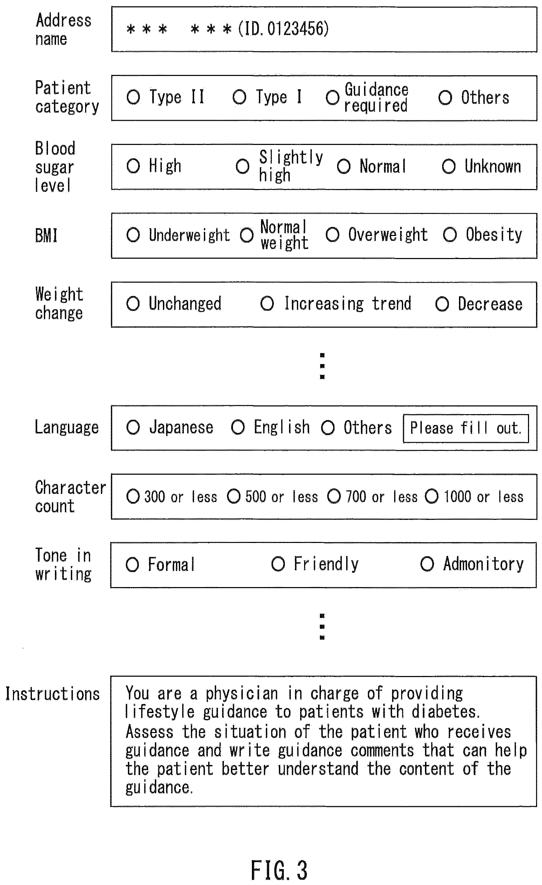

FIG. 3 is a diagram illustrating an example of a first information input screen that appears on the display screen of the display unit 50 when the physician in charge provides guidance comments to a diabetic patient by using the information input support system of this embodiment. The first information input screen assists the physician to enter data that will be acquired by the target person information acquisition portion and the text information acquisition portion.

As illustrated in FIG. 3, there is a set of options for each input item on the first information input screen, and the physician clicks or touches (when using a touch panel) an appropriate option to choose it. This can save the physician from having to type out necessary information.

The first information input screen contains the item of "address name" for entering the patient's name, ID, etc. to identify a diabetic patient who receives the guidance comments. The physician enters the patient's name, ID, etc. in a text box on the screen. As described above, when the input unit 60 functions as a bar code reader and reads information that identifies the target diabetic patient, the input patient information is displayed.

Moreover, as illustrated in FIG. 3, the information input support system 100 enables the physician to enter the personal information of the diabetic patient that is required for the guidance comments by only choosing an appropriate option from each of the items of, e.g., "patient category," "blood sugar level," "BMI," and "weight change" on the first information input screen. Specifically, the "patient category" item represents a classification of diabetes, the "blood sugar level" item represents a classification of the blood sugar level based on the measured data, the "BMI" item represents a classification of the degree of obesity, and the "weight change" item represents a classification of the weight change needed to determine the current condition of the diabetic patient.

As described above, when the diabetic patient uses an external measuring device (i.e., the target person information obtaining means) and the patient's biometric information (e.g., weight, blood sugar level, etc.) is input from the external measuring device through the input unit 60, or when the previously input data of the diabetic patient is stored in the storage unit 40, the selected items may be displayed by default, in each of which an appropriate option has been selected based on the target person data obtained from the input unit 60 or the storage unit 40. This allows the physician to confirm the input data, and also can save the physician the trouble of choosing an option.

The first information input screen also assists the physician to enter the text information data by presenting a set of options for each item in the text information data. These items relate to the formal aspects of the text in the guidance comments to be generated by the text generative AI.

As illustrated in FIG. 3, the information input support system 100 enables the physician to choose an appropriate option from each of the items of, e.g., "language," "character count," and "tone of writing" on the first information input screen. Specifically, the "language" item specifies the language used in the guidance comments by allowing the physician to choose or directly enter the intended language, the "character count" item specifies the number of characters in the guidance comments, and the "tone of writing" item specifies the tone of the text in the guidance comments.

The first information input screen contains the item of "instructions" that informs the text generative AI of a person who is considered to be a writer of the guidance comments. The physician can choose or enter a person who would write the guidance comments.

As described above, when the storage unit 40 previously stores the text information data and the data concerning the writer of the guidance comments, the data stored in the storage unit 40 may be displayed by default as the selected items on the information input screen. This can save the physician the trouble of entering data.

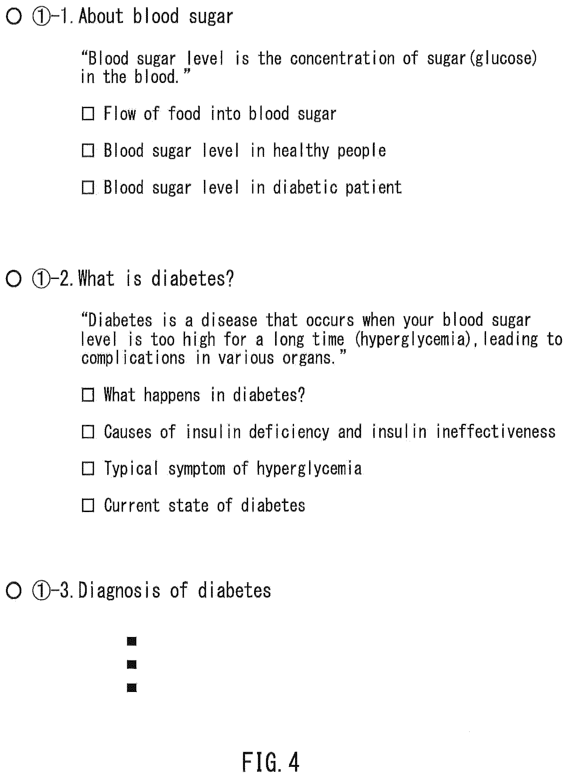

FIG. 4 is a diagram illustrating a second information input screen used for acquiring the guidance item data in the information input support system of this embodiment.

As illustrated in FIG. 4, the second information input screen assists the physician to enter guidance items, each indicating the content of guidance that is offered in the guidance comments. The second information input screen contains a list of guidance items defined by the Japan Association for Diabetes Education and Care (JADEC), and enables the physician to choose any of the guidance items suitable for the guidance comments to be prepared. The second information input screen is designed to prevent the physician from choosing a wrong guidance item. For example, when a guidance item is represented by "1-1 About blood sugar," as illustrated in FIG. 4, the second information input screen displays not only the outline of the guidance item such that "blood sugar level is the concentration of sugar (glucose) in the blood," but also the specific content of the guidance item, including "flow of food into blood sugar" and "blood sugar level in healthy people." As a result, the details of guidance are easier to understand, compared to the case where the second information input screen displays only the guidance items.

The specific contents of the guidance items are stored as text in the storage unit 40, which will be explained to a diabetic patient in response to each of the guidance items chosen by the physician on the second information input screen in FIG. 4. When the data acquisition unit 10 detects the selected guidance item, the specific content of the selected guidance item is transmitted as data from the storage unit 40. Further, this guidance item data is transmitted from the data acquisition unit 10 to the command creation unit 20, and thus can be reflected in the instruction commands given to the text generative AI.

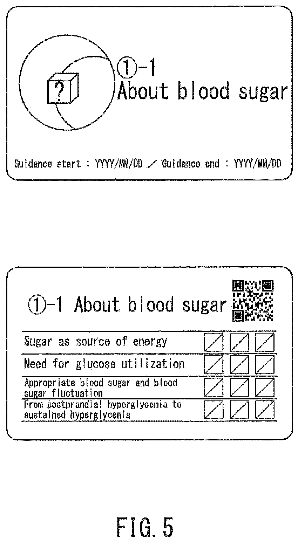

FIG. 5 is a diagram illustrating a medical care guidance card with a guidance item issued by the JADEC.

The medical care guidance card in FIG. 5 denotes the guidance item "1-1 About blood sugar" in FIG. 4. The front of the card is on the upper side of the figure and the back of the card is on the lower side of the figure.

The guidance comments for medical care in diabetes provided by using the information input support program in combination with the information input support system 100 of this embodiment are sent along with the medical care guidance card to a diabetic patient. This can help the diabetic patient better understand the guidance item while referring to the guidance comments and follow the corresponding guidance. Consequently, the medical care guidance for diabetes will be promoted.

There are fields to enter the date the diabetic patient receives guidance on the back of the medical care guidance card. Thus, both the physician and the diabetic patient can confirm the guidance history.

As described above, the requirements for generating the guidance comments are determined by the physician's choice from the input items in various types of data to be acquired by the data acquisition unit 10. Based on these requirements, the command creation unit 20 creates instruction commands that instruct the text generative AI to generate guidance comments.

When the text generative AI is, e.g., ChatGPT, the instruction commands may include the following: an "instruction statement" that indicates, e.g., preconditions for generating text and goals to be achieved by the generated text; and a series of commands starting with "##" that indicates specific conditions for generating text, e.g., "## language: Japanese," "## restriction: number of characters of 300 or less" (specifying restrictions on text generation), and "## context:" (specifying the required information in the text such as the content of guidance for the selected guidance item).

As described above, the specific content of the selected guidance item is given as a command starting with "## context" to ChatGPT. Therefore, ChatGPT will consider this context in creating text, which leads to guidance comments that reflect and highlight the specific content of the selected guidance item.

The instruction commands created by the command creation unit 20 are transmitted from the transmit-receive unit (instruction unit) 80 to the text generative AI in the server 300 through the Internet environment 200, and then the text generative AI generates guidance comments.

The AI-generated guidance comments are transmitted back to the information input support system 100 through the Internet environment 200 and displayed on the display unit 50. Therefore, the physician can check the AI's responses for any serious errors and send the resulting guidance comments to the diabetic patient either in electronic format or in paper format.

As described above, when the physician enters information that identifies the diabetic patient to write guidance comments for medical care in diabetes, the information input support system 100 assists the physician to enter the information that needs to be acquired by the text information acquisition portion 11, the target person information acquisition portion 12, and the guidance information acquisition portion 13 by presenting a set of options for each input item on the information input screen. The physician can choose an appropriate option from the set of options, thereby entering necessary information for the instruction commands given to the text generative AI to generate desired guidance comments. It is not necessarily essential to present options for all input items on the information input screen. As indicated by the item "language" in FIG. 3, if there are many possible options, some of them may be entered directly by the physician.

In the information input support system 100, the instruction commands created by the command creation unit 20 are transmitted from the transmit-receive unit 80 to the server 300 incorporating the text generative AI through the Internet environment 200, and the guidance comments generated by the text generative AI are received by the transmit-receive unit 80 and displayed on the display unit 50. Thus, the physician can check whether the guidance comments are appropriate or not before sending them to the diabetic patient.

In this embodiment, the data acquisition unit 10 includes the text information acquisition portion 11 that acquires the text information data indicating formal aspects of the text in the guidance comments. However, the text information acquisition portion 11 acquiring the text information data such as language, character count, and tone of writing is not essential, since the general text generative AI can automatically consider and generate text based on the input instruction commands.

This embodiment has been described, provided that the target person to be managed is a diabetic patient, the manager is a physician in charge of managing the patient's care and making guidance comments, and the management item is diabetes. However, the target person who receives the guidance comments generated by using the information input support system and the information input support program of embodiments of the present invention is not limited to a diabetic patient, but may include patients with various diseases (e.g., cardiovascular diseases such as hypertension and heart disease) for which the improvement of living conditions would be an important and effective treatment. The information input support system and the information input support program can also assist physicians in charge of these patients to enter necessary information for their guidance comments. In this case, the management item may vary depending on the disease the patient is suffering from.

In addition to assisting the physicians to provide the guidance comments to their patients, the information input support system and the information input support program of embodiments of the present invention also serve to assist health care advisers in entering data that allows the text generative AI to generate guidance comments. These AI-generated guidance comments will be provided with appropriate timing to those carrying out lifestyle improvement programs for health promotion and health care. In this case, the management item may be health care.

When the guidance comments are provided to the target people to be managed other than the diabetic patient, guidance items and their related guidance text issued by different medical organizations, or guidance items specifying basic guidance in health care programs may be used instead of the guidance items issued by the JADEC.

### Industrial Applicability

The information input support system and the information input support program of embodiments of the present invention are very useful in creating instruction commands that contain necessary and sufficient conditions for instructing the text generative AI to generate guidance comments, which are to be provided from the managers such as physicians and health care advisers to the target people to be managed such as patients and those carrying out health care programs.

The invention may be embodied in other forms without departing from the essential characteristics thereof, as defined by the appended claims. The embodiments disclosed in this application are to be considered in all respects as illustrative and not limiting. The scope of the invention is indicated by the appended claims rather than by the foregoing description, and all changes which come within the meaning and range of equivalency of the claims are intended to be embraced therein.

## Claims

1. An information input support system (100) that is configured to use text generative AI to generate guidance comments on a management item for a target person to be managed,
the information input support system comprising:
a data acquisition unit (10) that includes a target person information acquisition portion (12) that is configured to acquire target person data belonging to the target person to be managed, and a guidance information acquisition portion (13) that is configured to acquire guidance item data indicating a content of guidance that is offered in the guidance comments;
a command creation unit (20) that is configured to create instruction commands that instruct the text generative AI to generate text based on the target person data and the guidance item data that have been acquired by the data acquisition unit; and
an instruction unit (80) that is configured to provide the text generative AI with the instruction commands created by the command creation unit.

2. The information input support system according to claim 1, further comprising a storage unit (40) that is configured to store data concerning a plurality of guidance items that has been previously prepared,
wherein the guidance information acquisition portion (13) is configured to receive a selection of at least one guidance item from the previously prepared guidance items and acquire data concerning the at least one guidance item.

3. The information input support system according to claim 2, wherein the target person data has a plurality of items,
the storage unit (40) is configured to store a set of options that has been previously prepared for a predetermined item of the plurality of items of the target person data, and
the target person information acquisition portion (12) is configured to receive a selection of an option from the set of options and acquire the target person data corresponding to the predetermined item.

4. The information input support system according to any preceding claim, wherein the target person information acquisition portion (12) is configured to acquire the target person data from a target person information obtaining means for measuring or managing target person data belonging to the target person to be managed.

5. The information input support system according to any preceding claim, wherein the data acquisition unit (10) further includes a text information acquisition portion (11) that is configured to acquire text information data indicating formal aspects of the guidance comments to be generated by the text generative AI, and
the command creation unit (20) is configured to create the instruction commands based on the text information data.

6. The information input support system according to any preceding claim, wherein the command creation unit (20) is configured to create the instruction commands with priority given to the guidance item data acquired by the guidance information acquisition portion (13) of the data acquired by the data acquisition unit (10).

7. The information input support system according to any preceding claim, wherein the target person to be managed is a diabetic patient and the management item is diabetes.

8. An information input support program that allows text generative AI to generate guidance comments on a management item for a target person to be managed,
the information input support program comprising the step of:
creating instruction commands that instruct the text generative AI to generate the guidance comments based on target person data belonging to the target person to be managed and guidance item data indicating a content of guidance that is offered in the guidance comments, both the target person data and the guidance item data having been acquired by a data acquisition unit.
